# EUROPEAN PATENT APPLICATION

(11) **EP 3 839 059 A1**
(43) Date of publication of application: **23.06.2021**
(21) Application number: 19383118.7
(22) Date of filing: 16.12.2019
(51) Int. Cl.: C12Q 1/56, G01N 33/86, B01L 3/00

(54) **DEVICES AND METHODS FOR DETERMINING COAGULATION FACTOR ACTIVITIES**

(71) Applicant: CGT Enterprises, LLC, Laguna Niguel, CA 92677 (US)
(72) Inventor: GALMES SUREDA, Bernat, Laguna Niguel, CA 92677 (US); CANARO HIRNYK, Mariana Isabel, Laguna Niguel, CA 92677 (US); CORTINA GINER, Vicente R., Laguna Niguel, CA 92677 (US)
(74) Representative: HGF

(57) **Abstract**

Devices and methods for determining activity of one or more coagulation factors in a blood sample are provided. The device may comprise an inlet port for deposition of a sample, a reaction compartment, a detection compartment, a control compartment or any combination thereof. One or more compartments may be fluidically connected. One or more compartments may comprise plasma deficient of a coagulation factor, an ionic citrate source, an ionic calcium source, one or more coagulation contact phase activator reagents, a phospholipid or a mixture, or any combination thereof.

## Description

### BRIEF SUMMARY

An aspect of the disclosure provides a method. In some cases, the method comprises: (a) obtaining a blood sample from a subject; (b) adding a portion of the blood sample to a solution to form a mixture, wherein the solution comprises at least one Activated Partial Thromboplastin Time (aPTT) reagent, a salt solution, and a factor deficient plasma; (c) adding a calcium salt to the mixture; and (d) detecting coagulation of the blood sample. In some cases, the blood sample is whole blood. In some cases, the blood sample is blood plasma. In some cases, the portion of the blood sample is a non-diluted portion. In some cases, the factor deficient plasma is reconstituted. In some cases, the factor deficient plasma is depleted of at least one of: Factor II, Factor V, Factor VII, Factor VIII, Factor IX, Factor X, Factor XI, or Factor XII. In some cases, the factor deficient plasma is depleted of Factor VIII. In some cases, the subject has or is suspected of having a clotting disorder. In some cases, the clotting disorder is hemophilia. In some cases, the salt solution contains saline. In some cases, the aPTT reagent contains a silica acid, an ellagic acid, a phospholipid, or any combination thereof. In some cases, the calcium salt contains calcium chloride, calcium acetate, calcium carbonate, calcium glubionate, calcium gluconate, calcium hydroxide, calcium nitrate, calcium sulfonate, calcium phosphate, or a mixture of any one of these. In some cases, the calcium salt is a calcium chloride. In some cases, the detecting employs an imaging system.

Another aspect of the disclosure provides a cartridge. In some cases, the cartridge comprises: (a) a sample inlet port configured to receive a blood sample; (b) a mixing compartment configured to receive at least a portion of the blood sample from the sample inlet port, wherein the mixing compartment contains a depleted plasma that is depleted of a coagulation factor, wherein upon contact of the portion of the blood sample with the depleted plasma, a mixture forms; (c) a first reagent compartment that contains a calcium salt, wherein the mixing compartment is in fluidic communication with the first reagent compartment and is configured to receive a portion of the calcium salt from the first reagent compartment; and (d) a detection compartment configured to receive at least a portion of the mixture from the mixing compartment. In some cases, the cartridge further comprises a second reagent compartment that contains a coagulation contact phase activator, wherein the mixing compartment is in fluidic communication with the second reagent compartment and is configured to receive a portion of the coagulation contact phase activator from the second reagent compartment. In some cases, the first reagent compartment or the second reagent compartment are removable from the cartridge. In some cases, the first reagent compartment or the second reagent compartment further contains a citrate source, a tissue factor, a phospholipid, or any combination thereof. In some cases, the cartridge further comprises a capillary, wherein the capillary is configured to fluidically connect the mixing compartment with the first reagent compartment or the second reagent compartment. In some cases, the depleted plasma is lyophilized. In some cases, the depleted plasma is depleted of at least one of: Factor II, Factor V, Factor VII, Factor VIII, Factor IX, Factor X, Factor XI, or Factor XII. In some cases, the cartridge further comprises a reference compartment, wherein the reference compartment contains a reference sample. In some cases, the reference sample is a plasma containing the coagulation factor. In some cases, the reference sample is lyophilized. In some cases, the reference compartment is in fluidic communication with the first reagent compartment and is configured to receive at least a portion of the calcium salt from the first reagent compartment. In some cases, the calcium salt comprises calcium chloride, calcium acetate, calcium carbonate, calcium glubionate, calcium gluconate, calcium hydroxide, calcium nitrate, calcium sulfonate, calcium phosphate, or a mixture of any one of these. In some cases, the coagulation contact phase activator is a kaolin. In some cases, the coagulation contact phase activator is an ellagic acid. In some cases, the detection compartment is configured to communicate with a detector. In some cases, a system comprises the cartridge and a detector. In some cases, the detector is an imaging detector. In some cases, the cartridge is configured to communicate with the detector. In some cases, a kit comprises the cartridge, a sample collection tool, and instructions for use. In some cases, the kit further comprises an additional reagent compartment provided separately from the cartridge, the additional reagent compartment comprising the calcium salt, the coagulation contacts phase activator, or a combination thereof.

Another aspect of the disclosure provides a method. In some cases, the method comprises: (a) inputting a blood sample into a mixing compartment of a device; (b) mixing at least a portion of the blood sample with a depleted plasma that is depleted of a coagulation factor to form a mixture in the mixing compartment; (c) adding at least a portion of a citrate source, a coagulation contact phase activator, a tissue factor, a calcium salt, a phospholipid, or any combination thereof to the mixture; and (d) transferring at least a portion of the mixture to a detection compartment; and (e) detecting coagulation of the portion of the blood sample in the detection compartment. In some cases, the detecting employs an imaging-based detector. In some cases, the blood sample is whole blood. In some cases, the method further comprises detecting coagulation of a reference sample in a reference compartment. In some cases, the coagulation contact phase activator and the calcium salt are sequentially added to the mixing compartment. In some cases, the depleted plasma is depleted of at least one of: Factor II, Factor V, Factor VII, Factor VIII, Factor IX, Factor X, Factor XI, or Factor XII.

### INCORPORATION BY REFERENCE

All publications, patents, and patent applications mentioned in this specification are herein incorporated by reference to the same extent as if each individual publication, patent, or patent application was specifically and individually indicated to be incorporated by reference. To the extent publications and patents or patent applications incorporated by reference contradict the disclosure contained in the specification, the specification is intended to supersede and/or take precedence over any such contradictory material.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings (also "figure" and "FIG." herein), of which:
**FIG. 1** shows a computer control system that is programmed or otherwise configured to implement methods provided herein.
**FIG 2** is a diagram showing a method and system as disclosed herein.
**FIG 3** shows a bench top assay workflow for current clinical practice.
**FIG 4** shows components of a cartridge device as described herein.
**FIG 5** schematically illustrates a coagulation curve.
**FIG 6** schematically illustrates a coagulation curve.
**FIG 7** shows a first and second derivative coagulation times vs. log of Factor VIII.
**FIG 8** shows transmission across time.
**FIG 9** shows first and second derivative coagulation times vs. Factor VIII.
**FIG 10** shows first derivative across time.

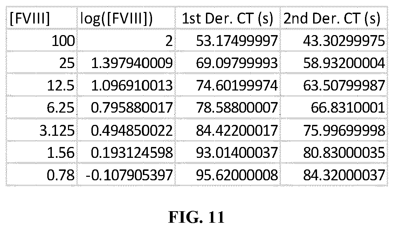

**FIG 11** shows a table of Factor VIII, log of Factor VIII, first and second derivatives.
**FIG 12** shows impact of hematocrit level on coagulation time, plotting coagulation time v. log of Factor VIII.
**FIG 13** shows the impact of hematocrit level on coagulation time, plotting absorbance v. wavelength.
**FIG 14** shows a test system including a cartridge device, an imaging system, and computer.
**FIG 15** shows a comparison of two different test setups, comparing dynamic range and signal to noise ratio (SNR).
**FIG 16** shows a main board design.
**FIG 17** shows a system block diagram.
**FIG 18** shows an OCI Now Development software (left side) and a cartridge device (right side).
**FIG 19** shows a keyed collar/socket for a two reagent vials.
**FIG 20** shows an example of a device.
**FIG 21A-B** shows coagulation measurements taken across time for varying concentrations of Factor VIII.

### DETAILED DESCRIPTION

While various embodiments of the invention have been shown and described herein, it will be obvious to those skilled in the art that such embodiments are provided by way of example only. Numerous variations, changes, and substitutions may occur to those skilled in the art without departing from the invention. It should be understood that various alternatives to the embodiments of the invention described herein may be employed.

Methods may include measuring a presence or absence of one or more factors in a blood sample. Methods may include diagnosing a blotting clotting disorder. Methods may include obtaining an sample, such as a whole blood sample, from a subject suspected of having a blood clotting disorder. A method may reduce the number of steps to perform the method. A method as described herein may comprise: (a) obtaining a blood sample from a subject; (b) adding a portion of said blood sample to a solution to form a mixture, wherein said solution comprises at least one Activated Partial Thromboplastin Time (aPTT) reagent, a salt solution, and a factor deficient plasma; (c) adding a calcium salt to said mixture; and (d) detecting coagulation of said blood sample. Methods may include measuring a hematocrit level or accounting for a known hematocrit level of a sample. Methods may include identifying a presence or absence of a co-factor, such as a level of vitamin K.

A device may be employed to measure a presence or absence of one or more factors in a sample, such as a whole blood sample. A device may be a cartridge device or a test strip. A system may comprise a device. The system may include a imager, a computer, a user interface, a heater, a pump, a database, a motor, or any combination thereof. A cartridge as described herein may comprise (a) a sample inlet port configured to receive a blood sample; (b) a mixing compartment configured to receive at least a portion of said blood sample from said sample inlet port, wherein said mixing compartment contains a depleted plasma that is depleted of a coagulation factor, wherein upon contact of said portion of said blood sample with said depleted plasma, a mixture forms; (c) a first reagent compartment that contains a calcium salt, wherein said mixing compartment is in fluidic communication with said first reagent compartment and is configured to receive a portion of said calcium salt from said first reagent compartment; and (d) a detection compartment configured to receive at least a portion of said mixture from said mixing compartment.

Hemophilia may affect an estimated 1 in every 5,000 births in the U.S. and approximately 20,000 Americans may be affected by Hemophilia A&B. A global population of patients with hemophilia may be more than 1,125,000. Morbidity and adverse events may be directly linked to insufficient factor VIII/IX level with children and adults experiencing 6 and 15 bleeding episodes annually. Approximately a third of Hemophilia A patients may visit the ER, of which 20% may be hospitalized due to a bleeding episode. Methods to quantify a factor (such as Factor VIII/IX) may require separation of plasma from whole blood sample and multiple analytical steps, that can only be carried out by professional laboratory staff with special laboratory equipment.

Methods as described herein, provide many improvements, including Point of Care (POC) testing with a drop of whole blood that can significantly improve diagnostics, patient outcome, and reduce cost. Methods as described herein may include a lab-on-chip device or a cartridge device. Devices may be configured to measurement one or more factors (including Factor VIII). Devices may be configured to assay small sample volumes, such as from about 1 uL of blood to about 20 uL of blood, or from about 1 uL of blood to about 15 uL of blood, or from about 1 uL of blood to about 10 uL of blood, or from about 1uL of blood to about 5uL of blood, or less than about 10uL of blood, or less than about 5uL of blood.

### Definitions

Unless otherwise indicated, open terms for example "contain," "containing," "include," "including," and the like mean comprising.

The singular forms "a", "an", and "the" are used herein to include plural references unless the context clearly dictates otherwise. Accordingly, unless the contrary is indicated, the numerical parameters set forth in this application are approximations that may vary depending upon the desired properties sought to be obtained by the present invention.

As used herein, the term "about" or "approximately" can mean within an acceptable error range for the particular value as determined by one of ordinary skill in the art, which will depend in part on how the value is measured or determined, e.g., the limitations of the measurement system. For example, "about" can mean plus or minus 10%, per the practice in the art. Alternatively, "about" can mean a range of plus or minus 20%, plus or minus 10%, plus or minus 5%, or plus or minus 1% of a given value. Alternatively, particularly with respect to biological systems or processes, the term can mean within an order of magnitude, within 5-fold, or within 2-fold, of a value. Where particular values are described in the application and claims, unless otherwise stated the term "about" meaning within an acceptable error range for the particular value should be assumed. Also, where ranges and/or subranges of values are provided, the ranges and/or subranges can include the endpoints of the ranges and/or subranges.

The term "substantially" as used herein can refer to a value approaching 100% of a given value. In some cases, the term can refer to an amount that can be at least about 90%, 91%, 92%, 93%, 94%, 95%, 96%, 97%, 98%, 99%, 99.9%, or 99.99% of a total amount. In some cases, the term can refer to an amount that can be about 100% of a total amount.

The term "sample" as used herein, generally refers to any sample of a subject (such as a blood sample). A sample may comprise a tissue, a cell, serum, plasma, exosomes, a bodily fluid, or any combination thereof. A bodily fluid may comprise blood, serum, plasma, urine, saliva, mucus, spinal fluid, tears, semen, bile, amniotic fluid, or any combination thereof. A sample or portion thereof may comprise an extracellular fluid obtained from a subject. A sample or portion thereof may comprise cell-free nucleic acid, DNA or RNA. A sample or portion thereof may be analyzed for a presence or absence or one or more Factors and in some cases, one or more co-factors. Genomic data may be obtained from the sample or portion thereof. A sample may be a sample suspected or confirmed of having a disease or condition. A sample may be a sample removed from a subject via a non-invasive technique, a minimally invasive technique, or an invasive technique. A sample or portion thereof may be obtained by a needle prick, a blood draw, or a combination thereof.
A sample may comprise whole blood. A sample may comprise plasma. In some cases, a blood sample may be centrifuged, such as prior to introducing the portion to a device. In some cases, a blood sample may not be centrifuged, such as prior to introducing the portion to the device. After centrifugation, a portion of plasma may be collected for use in a method as described herein.
A sample may be divided into one or more portions. A first portion may be added to a sample inlet of a device. A second portion may be added to a sample inlet of a second device or a second inlet port of the device. A portion may be stored for later use. A portion may be subjected to sequencing, imaging, an Eliza assay, or any other downstream application.

The term "subject," as used herein, may be any animal or living organism. A subject can be a mammalian subject. A subject can be a human subject. Subjects can be mammalian subjects, such as humans, non-human primates, rodents such as mice and rats, dogs, cats, pigs, sheep, rabbits, and others. Subjects can be fish, reptiles, or others. Subjects can be neonatal, infant, adolescent, or adult subjects. Humans can be more than about: 1, 2, 5, 10, 20, 30, 40, 50, 60, 65, 70, 75, or about 80 years of age. Humans can be from about 1 to about 5 years of age. Humans can be from about 1 to about 12 years of age. Humans can be from about 12 to about 20 years of age. Humans can be from about 18 to about 30 years of age. Humans can be from about 30 to about 50 years of age. Humans cam be from about 50 to about 70 years of age. The subject may have or be suspected of having a condition or a disease, such as a blood clotting disorder. The subject may be asymptomatic for a condition or a disease. The subject may be symptomatic for a condition or a disease. The subject may be a patient, such as a patient being treated for a condition or a disease, such as a blood clotting disorder patient. The subject may be predisposed to a risk of developing a condition or a disease such as a blood clotting disorder - such as based at least in part on a presence of a liver disease, low vitamin K, or an inherited genetic mutation. The subject may be healthy.

The term "blood coagulation reagent" as defined herein, relates to a compound or group of compounds, as well as to mixtures of compositions that allow coagulation of a blood sample alone or in combination with other reagents, said other reagents commonly being ionic calcium source (i.e., organic and inorganic calcium salts), as well as plasmas artificially depleted in any of the coagulation factors; coagulation contact phase activators, mixtures of tissue factor and phospholipids, and control samples including either blood or plasma comprising known amounts of any of the coagulation factors and covering factor activities considered both normal and pathologic values, according to commonly accepted reference ranges in hematology.

The term "coagulation contact phase activator" or "surface contact activators" (herein used interchangeably) relates to any compound that promotes activation of the intrinsic coagulation pathway by surface contact. This intrinsic pathway may also be referred to as contact activation pathway. The contact activation pathway begins with formation of the primary complex on collagen by high-molecular-weight kininogen (HMWK), prekallikrein, and FXII (Hageman factor). Prekallikrein is converted to kallikrein and FXII becomes FXIIa. FXIIa converts FXI into FXIa. Factor XIa activates FIX, which with its co-factor FVIIIa forms the tenase complex, which activates FX to FXa. The minor role that the contact activation pathway has in initiating clot formation can be illustrated by the fact that patients with severe deficiencies of FXII, HMWK, and prekallikrein do not have a bleeding disorder. Instead, the contact activation system seems to be more involved in inflammations. Examples of contact phase activators include, thus, any of the natural compounds or complexes formed with collagen, HMWK, and prekallikrein. Other examples of contact phase activators include artificial compounds of polyanionic nature, such as kaolin of general formula Al₂Si₂O₅(OH)₄, and other silicates, or organic acids, such as ellagic acid. These compounds are indeed mimics of cell surfaces or of tissue surfaces.

A "factor depleted plasma" refers to plasma (in some cases from an artificial origin) from which one or more target proteins have been removed, for example, by means of selective affinity immune-adsorption technologies or e.g. chemically. Generally, they are human citrated plasmas and they may be deficient plasmas in any of the following factors: II, V, VII, X, VIII, IX, XI, XII, or any combination thereof.

The expression "low levels of the coagulation factor to be measured" is to be understood as that the composition (i.e. plasma) has an amount of a specified coagulation factor lower than the amount considered as normal according to commonly accepted laboratory reference ranges. The amount of a particular coagulation factor in plasma can be determined both by non-functional (antigen) and functional (factor activity) means; both measures must not necessarily coincide and can be expressed as a percentage referred to by a primary international standard established by the World Health Organization (WHO). Focusing on factor activities, the factor present in a particular sample can be measured by comparing the value of some of the specific physical property that will vary as the clot is forming (which in turn will depend on the particular kind of assay used in the laboratory to determine the amount of factor, i.e. chromogenic, optical, electrical, etc.) with those of a calibration graph obtained from a calibrator with a known factor activity percentage referred to the WHO primary standard properly diluted to yield several calibration points and covering a factor activity percentage range broad enough to determine either normal and pathologic values, so that a particular variation in the physical property used to draft the calibration curve can be converted in a percentage of factor activity. Obviously, sample measures must be based on the same physical property, reagents, equipment and general procedure followed in the case of the calibration curve. Normal amounts of coagulation factors are those defined by a range of values including the values usually found in normal subjects. It is widely accepted that normal amounts (activities) may vary depending on the specific assay, reagents, equipment and procedure used to determine factor activities, among races and populations within said races, among the people with different blood groups, and so do the activity values of the coagulation factors. This is why the value of any of the coagulation factor activity varying typically from 70% to 150% could be considered normal. In the present description the expressions "amount/level of a coagulation factors" and "activity of a coagulation factor" are considered synonymous, because independently of the determination (non-functional vs. functional) one correlates with the other On the other hand, it is also widely accepted that a value of any of the coagulation factor activity varying from 40% to 70% is to be considered as non-normal but also non-pathologic; and that a value of any of the coagulation factor activity varying from 0% to 40% is considered non-normal and pathologic. A "pathological coagulation factor deficiency" refers to a deficiency degree of a particular factor implying a disease and/or with severe consequences for the maintenance of life.

### Factor Deficient Plasma

Factor deficient plasma may be deficient or substantially reduced in: Factor I, Factor II, Factor III, Factor IV, Factor V, Factor VI, Factor VII, Factor VIII, Factor IX, Factor X, Factor XI, Factor XII, Factor XIII, Factor XIV, Factor XV, Factor XVI, Factor XVII, Factor XVIII, Factor XIX, Factor XX or any combination thereof. Factor deficient plasma may be deficient in a first Factor and partially reduced in a second Factor. Factor deficient plasma may be deficient in more than one factor, such as deficient in two factors. Factor deficient plasma may be deficient in a factor for which a device may be detecting a presence or absent from a sample from a subject. For example, a subject is suspected of having a deficiency in Factor V and therefore, a device having a factor deficient plasma (that is deficient in Factor V) may be selected for analysis of a sample obtained from the subject. A device may comprise an amount of factor deficient plasma, such as a reaction compartment of a device. A factor deficient plasma may be formulated as a lyophilized pallet or otherwise immobilized to a specified location within a device, such as a reaction compartment.

### Factors

Blood or plasma (such as blood obtained from a subject) may be deficient or substantially reduced of one or more factors that may result in a blood clotting disorder. Blood or plasma may be deficient or substantially reduced of: Factor I, Factor II, Factor III, Factor IV, Factor V, Factor VI, Factor VII, Factor VIII, Factor IX, Factor X, Factor XI, Factor XII, Factor XIII, Factor XIV, Factor XV, Factor XVI, Factor XVII, Factor XVIII, Factor XIX, Factor XX or any combination thereof. Blood or plasma may be deficient or substantially reduced of: fibrinogen, prothrombin, tissue thromboplastin or tissue factor, ionized calcium (Ca++), labile factor or proaccelerin, stable factor or proconvertin, antihemophilic factor A or factor B, plasma thromboplastin component or Christmas factor, Stuart-Prower factor, plasma thromboplastin antecedent, Hageman factor, fibrin-stabilizing factor, prekalikerin, Fitzgerald factor, vWf, antithrombin III, heparin cofactor II, protein C, protein S, or any combination thereof.

Devices and methods as described herein detect a presence or an absence of one or more factors in a blood or plasma sample. In some cases, devices and methods detect a reduction in one or more factors in a blood or plasma sample. In some cases, devices and methods may also detect a presence or an absence of a co-factor, such as vitamin K. In some cases, devices and methods detect a presence or an absence of Factor II, Factor VII, Factor IX, Factor X, or any combination thereof and vitamin K, ionized calcium, a phospholipid, or any combination thereof. Devices and methods as described herein detect a presence or an absence of a factor implicated in an intrinsic pathway, extrinsic pathway, or both.

### Clotting Disorders

A clotting disorder may include: hemophilia A, hemophilia B, Factor deficiency (such as II, V, VII, X, or XII) or Von Willebrand's disease. A clotting disorder may result from: vitamin K deficiency, a low red blood cell count, a liver disease (cirrhosis, hepatitis, acute liver failure), or disseminated intravascular coagulation. A clotting disorder may be a hypercoagulable condition, such as an inherited or acquired condition.

An inherited hypercoagulable condition may include: Factor V Leiden; Prothrombin gene mutation; Deficiencies of natural proteins that prevent clotting (such as antithrombin, protein C and protein S); Elevated levels of homocysteine; Elevated levels of fibrinogen or dysfunctional fibrinogen (dysfibrinogenemia); Elevated levels of factor VIII and other factors including factor IX and XI; or Abnormal fibrinolytic system, including hypoplasminogenemia, dysplasminogenemia and elevation in levels of plasminogen activator inhibitor (PAI-1).

An acquired hypercoagulable condition may include: Cancer; Some medications used to treat cancer, such as tamoxifen, bevacizumab, thalidomide and lenalidomide; Recent trauma or surgery; Central venous catheter placement; Obesity; Pregnancy; Supplemental estrogen use, including oral contraceptive pills (birth control pills); Hormone replacement therapy; Prolonged bed rest or immobility; Heart attack, congestive heart failure, stroke and other illnesses that lead to decreased activity; Heparin-induced thrombocytopenia (decreased platelets in the blood due to heparin or low molecular weight heparin preparations); Lengthy airplane travel, also known as "economy class syndrome"; Antiphospholipid antibody syndrome; Previous history of deep vein thrombosis or pulmonary embolism; Myeloproliferative disorders such as polycythemia vera or essential thrombocytosis; Paroxysmal nocturnal hemoglobinuria; Inflammatory bowel syndrome; HIV/AIDS; or Nephrotic syndrome.

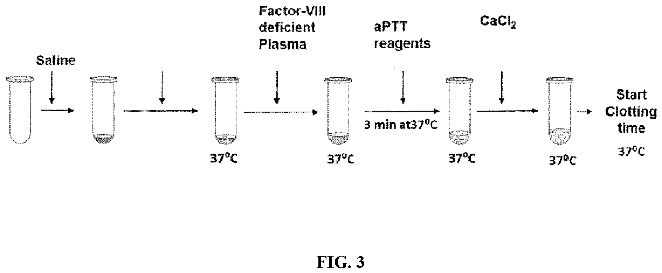

Referring to **FIG. 3****,** a current standard of care method may include (a) preparing a factor VIII deficient solution by reconstituting a lyophilized pellet with water; (b) prewarming saline, factor deficient plasma, aPTT, calcium chloride (CaCl₂) and empty tubes; (c) spinning down a whole blood sample to obtain a plasma sample; (d) metering the plasma sample and diluting the plasma sample with saline; (e) mixing the diluted plasma with factor VIII deficient plasma to form a mixture and incubating the mixture for 1 minute at 37 degrees Celsius; (f) adding aPTT to the mixture and incubating the mixture for 3 minutes at 37 degrees Celsius; (g) adding CaCh warmed to 37 degrees Celsius to the mixture; and (h) measuring a time to coagulation immediately.

As described herein, the number of steps of a method may be reduced. For example, an improved method may include (a) prewarming saline, aPTT, CaCh, and a cartridge or empty tubes; (b) mixing aPTT with a sample and factor VIII deficient plasma to form a mixture and incubating the mixture for 5 minutes at 37 degrees Celsius; (c) adding CaCl₂ to the mixture; and (d) measuring a time of coagulation. In some cases, saline and aPTT may be stored together, such as in a reagent compartment. In some cases, a mixture of saline and aPTT may be utilized to reconstitute factor-deficient plasma. In some cases, the method can be performed using a whole blood sample or a plasma sample.

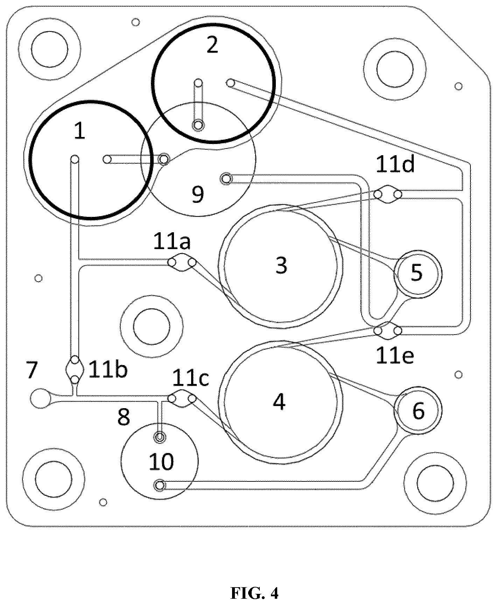

Referring to **FIG. 4****,** a device (such as a cartridge) may comprise one or more features, such as one or more: compartments, vents, valves, ports, or any combination thereof. A device may comprise a sample inlet port 7, such as a port configured to receive a blood sample or portion thereof. A device may comprise a blood metering channel **8** positioned between a sample inlet port and a reaction compartment **4.** A blood metering channel may be configured to meter a whole blood sample to from about 1uL to about 20 uL on the device. A blood metering channel **8** may comprise a channel length positioned between two valves **11b** and **11c.** A device may comprise one or more reaction compartments. A reaction compartment may comprise plasma deficient of one or more factors, such as plasma deficient of factor VIII. A device may comprise a control reaction compartment **3.** A device may comprise one or more detection compartments, such as **5** or **6.** A detection compartment may be separate from a reaction compartment or a control reaction compartment. A reaction compartment **4** may be fluidically connected to a detection compartment **6.** A control reaction compartment **3** may be fluidically connected to a detection compartment **5.** A device may comprise one or more air vents, such as **9** and **10.** A device may comprise one or more flow control valves, such as **11a, 11b, 11c, 11d,** and **11e.** A device may comprise one or more reagent compartments. For example, a device may comprise a first reagent compartment **1** and a second reagent compartment **2.** One or more reagent compartments may be removable from the device or provided separately and fluidically connected to the device. Reagent compartments may comprise one or more reagents. As shown in **FIG. 4****,** a first reagent compartment **1** may comprise saline and ATPP reagent mixture and a second reagent compartment **2** may comprise a calcium chloride solution. A reagent compartment may be fluidically connected to a reaction compartment, a control reaction compartment or a combination thereof. As shown in **FIG. 4****,** a first reagent compartment **1** may be fluidically connected to both the reaction compartment **4** and the control reaction compartment **3** and a second reagent compartment **2** may be fluidically connected to both the reaction compartment **4** and the control reaction compartment **3.**

### Sample Inlet Ports

A device may comprise a sample inlet port configured for receiving a sample, such as a plasma sample or a whole blood sample. A device may comprise more than one sample inlet port, for example, 2, 3, 4, 5, 6, 7 sample inlet ports or more. A sample inlet port may be configured to receive a blood sample, a plasma sample, or a portion thereof. A sample inlet port may be configured with a needle or a syringe. A sample inlet port may be configured to dilute the sample, such as diluting the sample with a buffer (such as water, salt solution, or other). A sample inlet port may not be configured to dilute the sample. A sample inlet port may be configured to filter a portion of the sample, such as filtering out at least a portion of red blood cells from the sample, debris, contaminants, or a combination thereof. A sample inlet port can be configured as a finger stick collection port. A sample inlet port may be configured as a pipet based collection port. A sample inlet port may comprise a cover to prevent contamination or other element (such as a seal) for reducing contamination. A sample inlet port may comprise a metering element to meter the blood sample received. A sample inlet port may be configured to receive a portion of a sample and send of a portion of the sample received to a reaction compartment. A sample inlet port may subdivide a sample and send one or more sub-portions to one or more reaction compartments.

### Reagent Compartments

A device may comprise one or more reagent compartments, such as 2, 3, 4, 5, 6, 7, 8, 9, 10 reagent compartments or more. A reagent compartment may be provided separately from a device. A reagent compartment may be formulated as part of the device. A reagent compartment may be fluidically connected to one or more reaction compartment, control reaction compartment, detection compartment, or any combination thereof. In some cases, a flow path forms from a reagent compartment to a reaction compartment to a detection compartment. In some cases, a flow path forms from a reagent compartment to a reaction compartment. In some cases, a flow path forms from a reagent compartment to a control reaction compartment. In some cases, a flow path forms from a first reagent compartment to a reaction compartment and a second flow path forms from a second reagent compartment to the reaction compartment.

A reagent compartment may comprise a calcium salt. A reagent compartment may comprise calcium chloride, calcium acetate, calcium carbonate, calcium glubionate, calcium gluconate, calcium hydroxide, calcium nitrate, calcium sulfonate, calcium phosphate, or a mixture of any one of these. A reagent compartment may comprise calcium chloride. A reagent compartment may comprise an activated partial thromboplastin time (aPTT). A reagent compartment may comprise an aPTT and a salt solution (such as a saline). A reagent compartment may comprise a silica acid, an ellagic acid, a phospholipid, or any combination thereof. A reagent compartment may comprise a citrate source, a tissue factor, a phospholipid, or any combination thereof. A reagent compartment may comprise a coagulation contact phase activator (such as kaolin or ellagic acid).

### Reaction Compartments

A device (such as a cartridge device) may comprise a reaction compartment. In some cases, a device may comprise more than one reaction compartment, such as 2, 3, 4, 5, 6, 7, 8, 9, 10 or more. In some cases, a reaction compartment may be a control reaction compartment. A reaction compartment may comprise a stir bar or other element for mixing the contents. A reaction compartment may comprise a factor deficient plasma. A reaction compartment may not comprise a factor deficient plasma. The factor deficient plasma may be formulated such that it substantially remains in the reaction compartment prior to initiating an assay. The factor deficient plasma may be formulated as a pallet. The factor deficient plasma may be lyophilized. The factor deficient plasma may be deficient of one or more factors. The factor deficient plasma may be substantially reduced of one or more factors.

A reaction compartment may be configured to receive a sample from one or more sample inlets. A reaction compartment may be configured to receive a reagent from one or more reagent compartments. A reaction compartment may be configured to send at least a portion of its contents to one or more detection compartments.
A reaction compartment may comprise a plasma deficient of more than one factor. A reaction compartment may comprise a plasma deficient of a single factor. A device may comprise multiple reaction compartments, and a first reaction compartment may comprise a plasma deficient of a first factor and a second reaction compartment may comprise a plasma deficient of a second factor.

### Detection Compartments

A device may comprise a detection compartment. In some cases, a device may comprise more than one detection compartment, such as 2, 3, 4, 5, 6, 7, 8, 9, 10 or more. A reaction compartment may comprise the detection compartment. A detection compartment may be separate from a reaction compartment. A detection compartment may be configured to receive as least a portion of a mixture formed in a reaction compartment. A detection compartment may be operatively linked to a detector, such as an imaging system. A detection compartment may be configured for acquisition of one or more images of at least a portion of the detection compartment. A device may comprise a detection compartment corresponding to a test sample and a detection compartment corresponding to a control sample. A detection compartment may be positioned on a horizontal axis that is different than a horizontal axis on which a reaction compartment may be positioned.

### Vents

A device may comprise a vent. A device may comprise more than one vent, such as 2, 3, 4, 5, 6, 7, 8, 9, 10 or more. A vent may be configured to accommodate an overflow of fluid, such as an overflow of a blood sample, a reagent, a waste product, or any combination thereof. A vent may be positioned proximal a reagent compartment, a sample inlet port, a reaction compartment, a detection compartment, or any combination thereof. A vent may be configured to introduce air into the device, to permit flow of a fluid within the device, or a combination thereof.

### Valves

A device may comprise a valve. A device may comprise more than one valve, such as 2, 3, 4, 5, 6, 7, 8, 9, 10 or more. A valve may be a pneumatic control valve. A valve may be positioned proximal a reagent compartment, a sample inlet port, a reaction compartment, a detection compartment, or any combination thereof. A valve may be positioned in a flow stream between a reagent compartment and a sample inlet port or reaction compartment. A valve may be positioned in a flow stream between a reaction compartment and a reagent compartment. A valve may be a pneumatic valve. Flow of fluid within a device may be driven by air entering to a compartment that may force the fluid out of the compartment, such as a reagent compartment. In some cases, pressure may be applied to the compartment. A valve may control backflow of a fluid.

### Kits

A kit may include a device as described herein. A device may be configured as a cartridge. A device may be configured as a test strip. A device or portion thereof may be reusable. A device or portion thereof may be configured for single use. One or more reaction compartments may be provided separately from the device as part of a kit and fluidically connected to a portion of the device before use. A kit may include the device and one or more compartments provided separately (such as a reaction compartment). A kit may include instructions for use. A kit may include one or more sample collection tools, such as a needle, syringe, pipette, container, or any combination thereof. A kit may include one or more reagents, such as one or more reagents provided in one or more containers, in a compartment of a device (such as a reaction compartment), or a combination thereof. A kit may include factor deficient plasma provided in one or more containers, in a reaction compartment of a device, or combination thereof. A kit may include a detection system, such as an imaging system. A kit may include an algorithm for analyzing a result obtained from one of the methods as described herein. A kit may comprise reference values or a database of results. A kit may include one or more controls, such as a control blood sample. A control may be provided separately from a device or may be provided within a compartment of the device, such as a control reaction compartment.

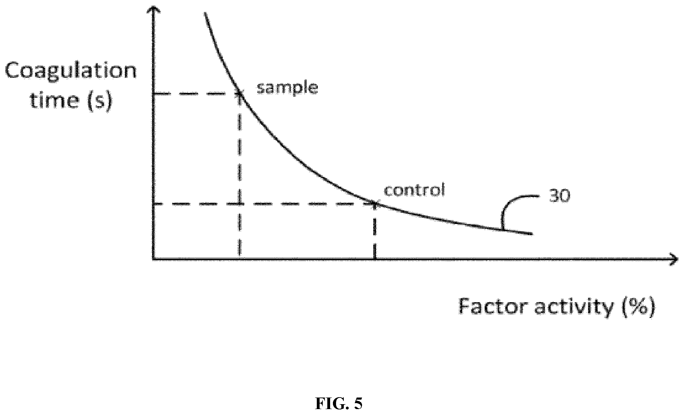

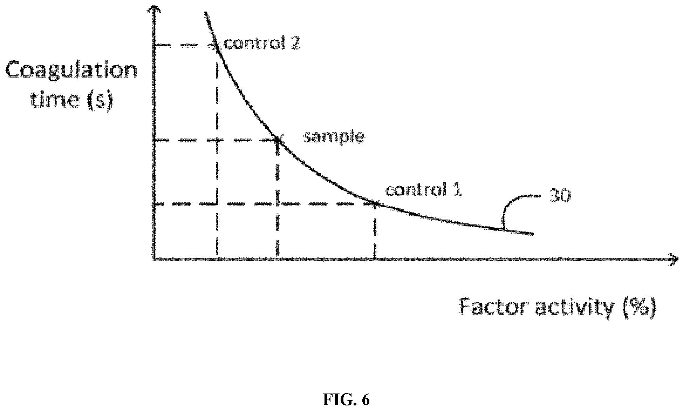

Referring to **FIG. 5** and **FIG. 6****,** a device may comprise one or more control compartments. A control reaction compartment may comprise a mixture of blood or plasma with a known amount of coagulation factor, a citrate source, one or more contact phase activator reagents and phospholipids, an ionic calcium source, or any combination thereof. A device may be suitable for factors involved in an intrinsic pathway. In some cases, a device may be more suitable for an extrinsic pathway factors, such as when a control reaction compartment comprises a mixture of blood or plasma with a known amount of coagulation factor, a citrate source, an ionic calcium source, a mixture of tissue factor and phospholipids or any combination thereof. A control reaction compartment may serve to check or confirm one or more results obtained in the reaction compartment or detection compartment. Referring to **FIG. 5** and **FIG. 6****,** a coagulation curve **30** is showing a relation between coagulation time and a level of activity of a specific coagulation factor.

Methods as described herein may include combining a salt buffer (such as saline) with aPTT. In some cases, a mixture of saline and aPTT can be stored together. The mixture of salt buffer and aPTT may be directly added to a sample obtained from a subject, such as a whole blood sample or plasma sample. The sample from the subject may be diluted or may not be diluted. The mixture of aPTT and salt buffer may be added to the sample obtained from the subject and then the mixture may be utilized to reconstitute a factor deficient plasma. In some cases, the mixture of aPTT and salt buffer may be added to reconstitute the factor deficient plasma before the sample obtained from the subject is engaged. Factor deficient plasma may be lypophilized, may be a solid or a semi-solid, or may be freeze dried. aPTT may be a reagent that initiates a coagulation process, an activator of coagulation, or a platelet phospholipid substitute. Here, aPTT may be added to reconstitute the factor deficient plasma and the aPTT may be mixed with a salt buffer. In some cases, the aPTT may be added towards the end of the method, such as immediately before adding the calcium source. The method may not require a centrifugation step. The method may provide a reduced number of steps, such as mixing steps. In some cases, the sample from the subject may not be diluted. In some cases, the aPTT and saline mixture may be added to reconstitute the factor deficient plasma. In some cases, the aPTT and saline mixture may be added after reconstitution of factor deficient plasma. A first reagent compartment may comprise aPTT and saline.

In some cases, a mixture of aPTT and salt buffer (from a reagent compartment) is mixed with a sample from a subject (such as a undiluted sample) and the mixture is utilized to reconstitute a dried factor deficient plasma (such as in a reaction compartment). A calcium source may be added to the mixture in the reaction compartment, such as adding a calcium source from a separate reagent compartment. A portion of the mixture (from a reaction compartment) may flow to a detection compartment.

A detection compartment may be configured to receive a portion of a mixture from a reaction compartment. The mixture may comprise whole blood. The mixture may comprise red blood cells. Detection may be performed by an imager. Detection may comprise measuring an absorbance of the mixture. Opacity may correlate with coagulation. A control detection compartment may comprise one or more reagents, factor deficient plasma, and may not comprise a sample. A detection compartment may comprise one or more reagents, factor deficient plasma, and the sample, such as a blood sample.

Adding a reagent from the reagent compartment to a blood sample received from the sample inlet port may permit dilution of the blood sample.

A device may be a diagnostic device. A device may be utilized for diagnosing a clotting disorder, such as hemophilia. A device may be utilized for diagnosing a secondary condition such as a liver disease or vitamin K deficiency. A device may diagnose a clotting disorder with at least about 80%, 85%, 90%, 95% accuracy. A device may diagnose a clotting disorder with at least about: 80%, 85%, 90%, 95% specificity. A device may diagnose a clothing disorder with at least about: 80%, 85%, 90%, 95% sensitivity. A device may measure a presence or absence of one or more factors. A device may measure a level of one or more factors, such as a reduced level that provides a pathological outcome. A device may measure a presence or absence of one or more co-factors, such as vitamin K. A device may measure a level of one or more co-factors. A device may measure a presence or absence of one or more genetic mutations or variations of one or more genes. A device may measure an expression level of one or more genetic mutations or variations of one or more genes. A result of a measurement may inform a diagnosis of a subject. The subject may be suspected of having a condition, such as a blood clotting condition. The subject may be at risk of developing the condition. The subject may have the condition. The subject may be symptomatic or asymptomatic for the condition. A device may be configured for home use, field use, or clinical use. A subject may utilize a device in a home setting. A medical professional may utilize the device in a medical setting.

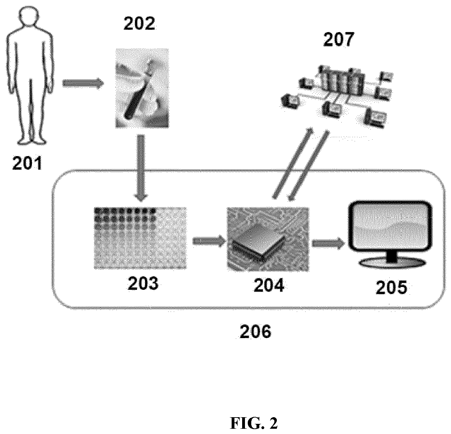

Referring to **FIG. 2****,** a sample **202** containing blood or plasma may be obtained from a subject **201,** such as a human subject. A sample **202** may be subjected to one or more methods as described herein, such as performing an assay. In some cases, an assay may comprise a coagulation assay using a device as described herein. One or more results from a method may be input into a processor **204.** One or more input parameters such as a sample identification, subject identification, sample type, a reference, or other information may be input into a processor **204.** One or more metrics from an assay may be input into a processor **204** such that the processor may produce a result, such as a diagnosis of a blood clotting disorder or a recommendation for a treatment. A processor may send a result, an input parameter, a metric, a reference, or any combination thereof to a display **205,** such as a visual display or graphical user interface. A processor **204** may (i) send a result, an input parameter, a metric, or any combination thereof to a server **207,** (ii) receive a result, an input parameter, a metric, or any combination thereof from a server **207,** (iii) or a combination thereof.

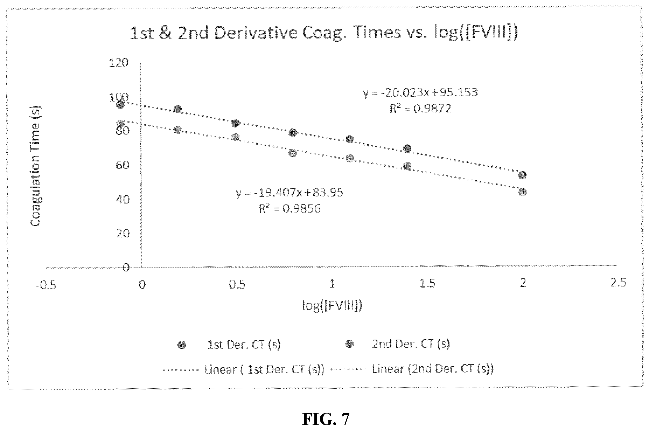

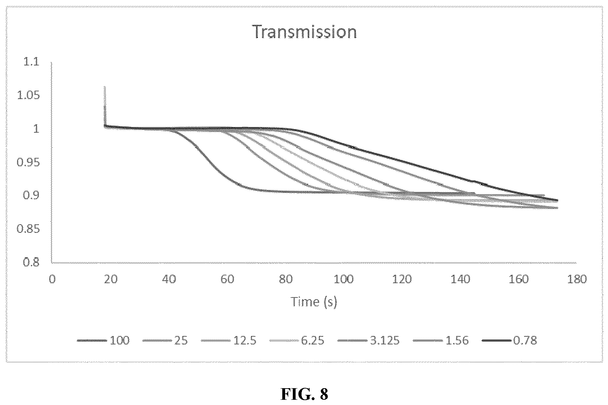

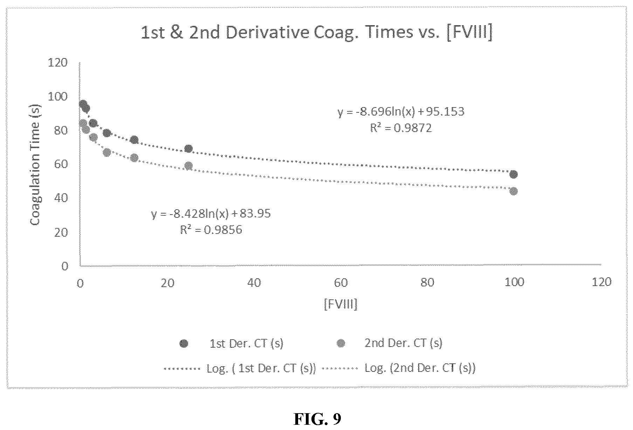

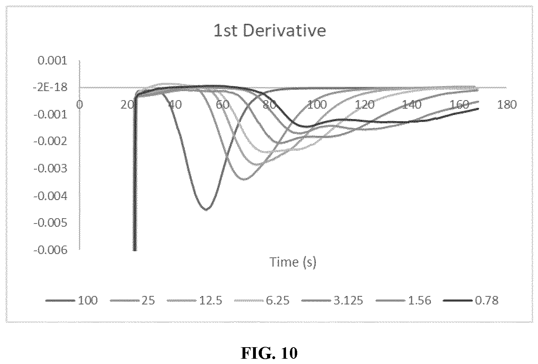

Referring to **FIG 7****-** **FIG 10****,** coagulation times can be plotted in various formats. For example, in **FIG 7****,** a first and second derivative coagulation times is plotted against a log of a factor (Factor VIII). **FIG 8** shows transmission across time. **FIG 9** shows first, and second derivative coagulation times plotted against a factor (Factor VIII). **FIG 10** shows first derivative across time.

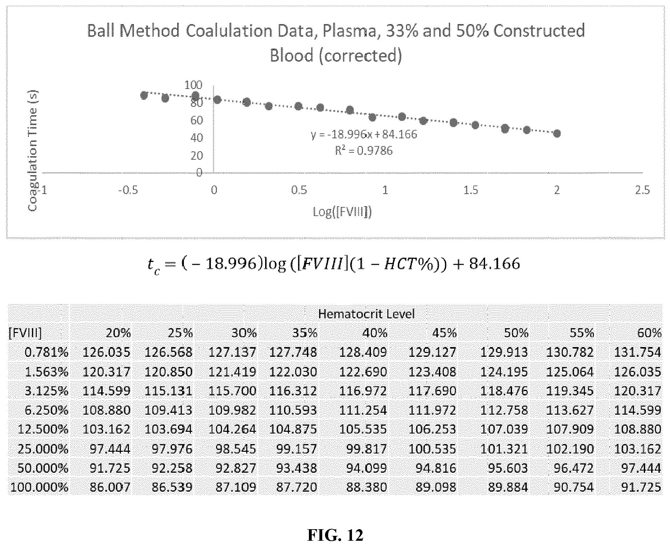

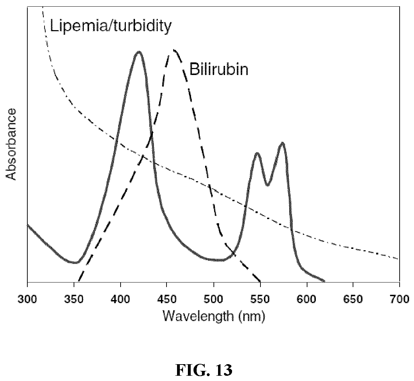

Referring to **FIG. 12****,** the level of hematocrit may impact coagulation time independent of the amount of factor present in a sample. **FIG. 12** shows the impact of hematocrit level on coagulation time by plotting coagulation time v. log of factor (in this case Factor VIII). This relationship is also represented by the numerical values below the graphical representation. A result may be modified or updated according to a hematocrit level present in the sample. In some cases, a hematocrit level may not impact coagulation time. In some cases, a hematocrit level may impact coagulation time. For example, from about 1% to about 20%, from about 1% to about 25%, from about 1% to about 10% hematocrit - may not substantially alter a coagulation time. From about 20% to about 60%, from about 15% to about 60%, 20% or more, 30% or more, 40% or more, 50% or more hematocrit - a coagulation time may be substantially altered. Hematocrit level may impact coagulation time from about 0.1 to about 0.2 second/% hematocrit; from about 0.1 to about 0.5 second/% hematocrit; from about 0.1 to about 1 second/% hematocrit. A device may be configured to measure hematocrit level. A device may interface with a detector to detect a hematocrit level. Detection may be image based. Detection of coagulation and detection of hematocrit may be a single measurement, performed in a detection compartment. Detection of coagulation and detection of hematocrit may be measured separately. A method may be configured to account for hematocrit level in producing a result. Referring to **FIG. 13****,** it may be possible to approximately measure hematocrit level by measuring light transmission, such as with green or red LED.

A device or system may measure hematocrit, % of blood cells in whole blood, in a sample, such as a whole blood sample. A level of hematocrit may vary from subject to subject. A level of hematocrit may influence a coagulation time independent of an amount of one or more factors present in a sample. A method may include determined a hematocrit correction factor and applying the hematocrit correction factor to a result. Coagulation may be measured by an optical method, such as absorbance. Coagulation may be measured by a steel ball method. In this method, a magnetic ball may be moved using gravity and when the ball deflects more than it should - then coagulation may have started. This measurement method is an alternative to an optical based method of measuring coagulation. In some cases, time to coagulation may change with hematocrit when having the same amount of factor. A user may provide a hematocrit level. A plasma sample may be utilized to set a standard curve for level of hematocrit to coagulation time. Bilirubin may also influence an absorbance measurement and like hematocrit, may be factored out.

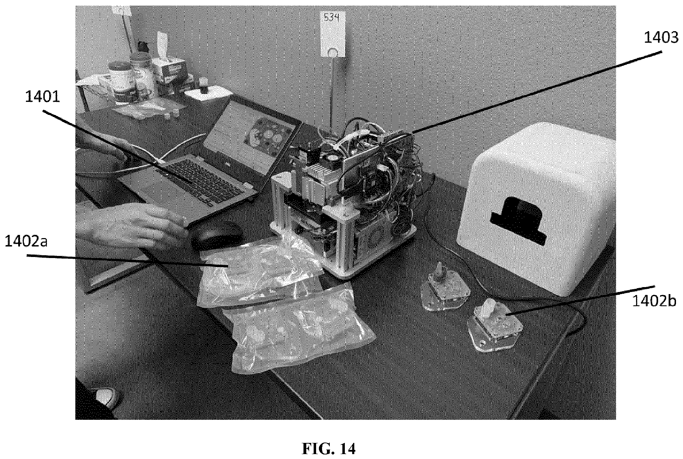

Referring to **FIG. 14****,** shows a test setup. A cartridge device, including two reagent compartments in shown wrapped in packaging in 1402a and opened from the packaging in 1402b. A cartridge device can be mounted onto a platform 1403, such that the coagulation assay can be performed and a coagulation time, hematocrit level, a co-factor measurement, or any combination thereof can be measured. A computer 1401 can interface with the device and platform to direct performance of the assay and acquisition of the one or more measurements.

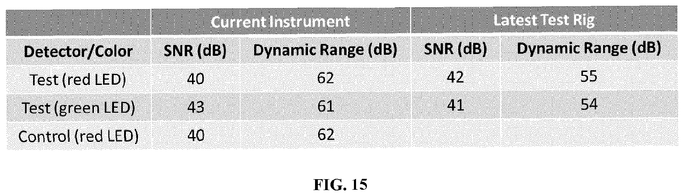

Referring to **FIG. 15****,** a sensitivity comparison was performed between two devices "current instrument" and "latest test rig" to show Signal to Noise Ratio (SNR) and dynamic range. Power level was reduced to about 63% for "current instrument" red LED tests to approximately match the same output level as existing data from "latest test rig". With better tuning of the LED fan control, SNR may likely improve.

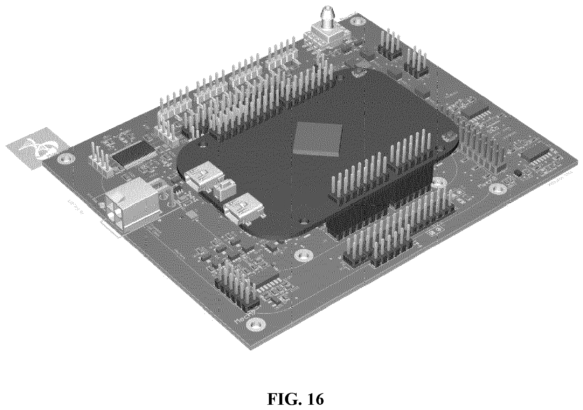

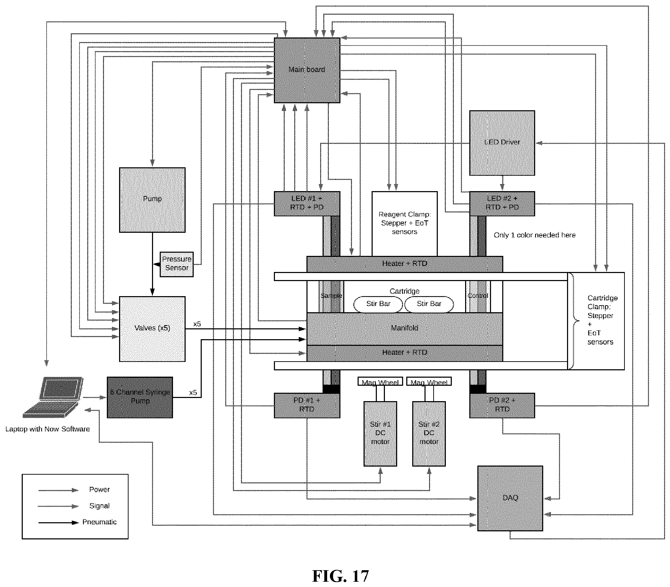

A main board design is shown in **FIG 16** and a system block diagram is shown in **FIG 17** including the system elements of a DAQ, 2 DC motors to drive a stir bar in a reaction compartment, a cartridge clamp, EoT sensors, two LED (#1 and #2) and LED driver, two stir bars, two heating elements, a manifold, a pump, a pressure sensor, five valves, 6 channel syringe pumps, a main board, and laptop with software. Red lines indicate power, blue lines indicate signal transmission, and black lines indicate pneumatic function.

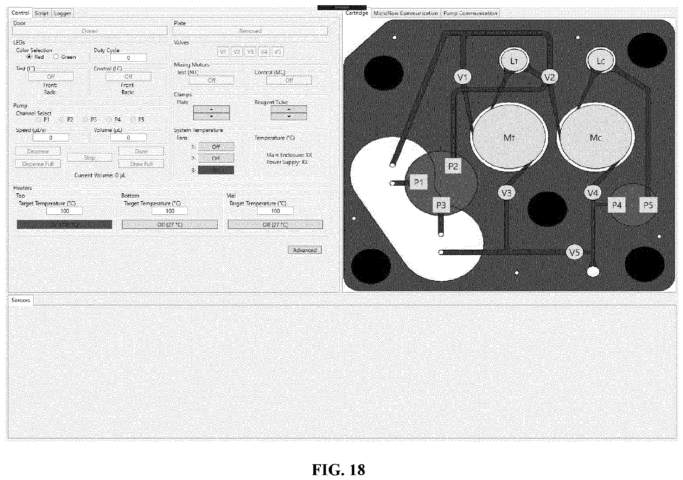

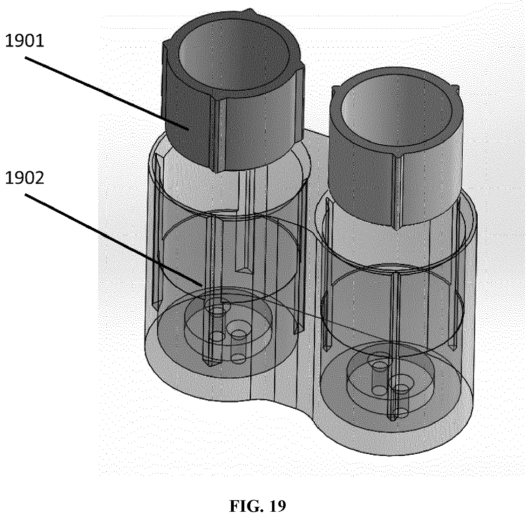

**FIG 18** shows an OCI Now Development software (left side) and a cartridge device (right side). The cartridge device provides one example of how the vents, valves, and compartments may be positioned and interact with one another. For example, P₁, P₂, P₃, P₄, and P₅ can be a pump. V₁, V₂, V₃, V₄, V₅ can be valves. M_{T} can be the reaction compartment configured to receive the test blood sample. Mc can be the control reaction compartment. L_{T} can be the detection compartment configured to receive a portion of the test blood sample from the reaction compartment. Lc can be the detection compartment configured to receive a portion of fluid from the control reaction compartment. **FIG. 19** shows a keyed collar/socket 1901 for a one of two reagent vials 1902.

A sample can be introduced to a cartridge device at a position closed to V₄ and V₅ on **FIG. 18****.** The sample can be metered, for example, by a semi-permeable membrane P4 that may block the sample from moving further.

A cartridge port may be sealed. V indicated a valve. P indicates a pump action. Valves may be controlled by a pressure source that may be open or closed to valve. A valve may be defaulted to open and when a pressure is applied it closes. Pressure may be generated by a diaphragm, such as a diaphragm within the device. A pump may be generated by a single syringe pump. Diaphragm pump may control pressure.

One or more interfaces may be sealed, and valves initially may be closed. When a sample is applied, it may be added in an excess of from about 10 uL to about 40 uL. A sample may be drawn into a channel of the device by P4 membrane. This may be an example of how the blood metering occurs.

A cap may be off at an inlet and can act as a valve. Then 10 uL of the sample can be metered into the reaction chamber. This can be done by opening V5 and V4 and pushing from P3. P3 can pushes air into one of the reagent packs and the sample can get pushed back out.

aPTT and saline reagent may be pushed into Mc. Using P3, a sample may be pushed into Mc. Then mixer can turn on and off and a sample can be incubated in a reaction compartment, for about 5 minutes.

P₁ can push CaCl into Mc from a second partition of a reagent pack. After about 10 secs of mixing CaCl, a sample can be moved to a detection compartment. Microbubbles from a stir bar located in a reaction compartment may affect accuracy of detection, such as an absorbance based detection. Additionally, blood absorbance may be high and to collect an accurate measurement a very thin chamber for light to pass through may be desirable. Detection may be acquired by LED.
At time zero, CaCl can be introduced and at time 20, can be moved to a detection compartment. A measurement can be normalized. Coagulation may happen when inflection of transmission begins. ACL Top can be a reference selected. ACL Top can look at absorbance as coagulation happens.
A maximum of inflection may indicate a point of coagulation. A transmission plot may provide a stable line across time followed by a change or inflection point - indicating coagulation.

Methods may include performing an assessment of sensitivity and dynamic range of measuring coagulation time. Methods may include calibrations, such as ensuring one or more optics of a detector are aligned to a device or ensuring fluidics components do not leak or fail to maintain appropriate flow or pressure. Methods may include heating a device or maintaining a constant temperature and devices may comprise a heating element or operatively connect to a heating element. A device may interface with a pump, such as a syringe pump. A computer system may direct the pump. A system may comprise a device and a platform, such as a user interface. The user interface may be connected to one or more systems such as a syringe pump, DAQ, or others. One or more reagent compartments may comprise keyed collar / socket design for reagent vials that interface with the device. A device may comprise one or more clamps (such as a cartridge or reagent clamp), heating block, cartridge port door, optics, or any combination thereof.

### Computer control systems

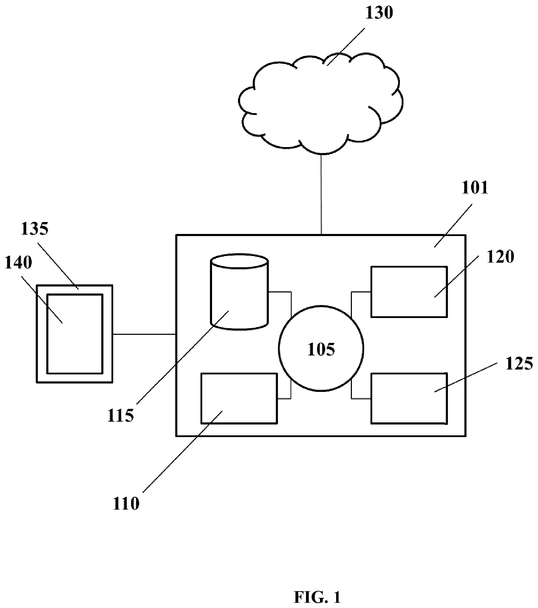

The present disclosure provides computer control systems that are programmed to implement methods of the disclosure. **FIG. 1** shows a computer system 101 that is programmed or otherwise configured to control one or more valves and vents of a device, control flow of fluid within the device, control communication between the device and an imager, control analysis of a result obtained from the device, or any combination thereof. The computer system 101 can regulate various aspects of a device operation, result analysis, interfacing with an imager, or a method of the present disclosure. The computer system 101 can be an electronic device of a user or a computer system that is remotely located with respect to the electronic device. The electronic device can be a mobile electronic device.

The computer system 101 includes a central processing unit (CPU, also "processor" and "computer processor" herein) 105, which can be a single core or multi core processor, or a plurality of processors for parallel processing. The computer system 101 also includes memory or memory location 110 (e.g., random-access memory, read-only memory, flash memory), electronic storage unit 115 (e.g., hard disk), communication interface 120 (e.g., network adapter) for communicating with one or more other systems, and peripheral devices 125, such as cache, other memory, data storage and/or electronic display adapters. The memory 110, storage unit 115, interface 120 and peripheral devices 125 are in communication with the CPU 105 through a communication bus (solid lines), such as a motherboard. The storage unit 115 can be a data storage unit (or data repository) for storing data. The computer system 101 can be operatively coupled to a computer network ("network") 130 with the aid of the communication interface 120. The network 130 can be the Internet, an internet and/or extranet, or an intranet and/or extranet that is in communication with the Internet. The network 130 in some cases is a telecommunication and/or data network. The network 130 can include one or more computer servers, which can enable distributed computing, such as cloud computing. The network 130, in some cases with the aid of the computer system 101, can implement a peer-to-peer network, which may enable devices coupled to the computer system 101 to behave as a client or a server.

The CPU 1105 can execute a sequence of machine-readable instructions, which can be embodied in a program or software. The instructions may be stored in a memory location, such as the memory 110. The instructions can be directed to the CPU 105, which can subsequently program or otherwise configure the CPU 105 to implement methods of the present disclosure. Examples of operations performed by the CPU 105 can include fetch, decode, execute, and writeback.

The CPU 105 can be part of a circuit, such as an integrated circuit. One or more other components of the system 101 can be included in the circuit. In some cases, the circuit is an application specific integrated circuit (ASIC).

The storage unit 115 can store files, such as drivers, libraries and saved programs. The storage unit 115 can store user data, e.g., user preferences and user programs. The computer system 101 in some cases can include one or more additional data storage units that are external to the computer system 101, such as located on a remote server that is in communication with the computer system 101 through an intranet or the Internet.

The computer system 101 can communicate with one or more remote computer systems through the network 130. For instance, the computer system 101 can communicate with a remote computer system of a user (e.g., a computer, tablet, smart phone, server, or other). Examples of remote computer systems include personal computers (e.g., portable PC), slate or tablet PC's (e.g., Apple® iPad, Samsung® Galaxy Tab), telephones, Smart phones (e.g., Apple® iPhone, Android-enabled device, Blackberry®), or personal digital assistants. The user can access the computer system 101 via the network 130.

Methods as described herein can be implemented by way of machine (e.g., computer processor) executable code stored on an electronic storage location of the computer system 101, such as, for example, on the memory 110 or electronic storage unit 115. The machine executable or machine readable code can be provided in the form of software. During use, the code can be executed by the processor 105. In some cases, the code can be retrieved from the storage unit 1115 and stored on the memory 110 for ready access by the processor 105. In some situations, the electronic storage unit 115 can be precluded, and machine-executable instructions are stored on memory 110.

The code can be pre-compiled and configured for use with a machine having a processer adapted to execute the code or can be compiled during runtime. The code can be supplied in a programming language that can be selected to enable the code to execute in a pre-compiled or as-compiled fashion.

Aspects of the systems and methods provided herein, such as the computer system 101, can be embodied in programming. Various aspects of the technology may be thought of as "products" or "articles of manufacture" typically in the form of machine (or processor) executable code and/or associated data that is carried on or embodied in a type of machine readable medium. Machine-executable code can be stored on an electronic storage unit, such as memory (e.g., read-only memory, random-access memory, flash memory) or a hard disk. "Storage" type media can include any or all of the tangible memory of the computers, processors or the like, or associated modules thereof, such as various semiconductor memories, tape drives, disk drives and the like, which may provide non-transitory storage at any time for the software programming. All or portions of the software may at times be communicated through the Internet or various other telecommunication networks. Such communications, for example, may enable loading of the software from one computer or processor into another, for example, from a management server or host computer into the computer platform of an application server. Thus, another type of media that may bear the software elements includes optical, electrical and electromagnetic waves, such as used across physical interfaces between local devices, through wired and optical landline networks and over various air-links. The physical elements that carry such waves, such as wired or wireless links, optical links or the like, also may be considered as media bearing the software. As used herein, unless restricted to non-transitory, tangible "storage" media, terms such as computer or machine "readable medium" refer to any medium that participates in providing instructions to a processor for execution.

Hence, a machine readable medium, such as computer-executable code, may take many forms, including but not limited to, a tangible storage medium, a carrier wave medium or physical transmission medium. Non-volatile storage media include, for example, optical or magnetic disks, such as any of the storage devices in any computer(s) or the like, such as may be used to implement the databases, etc. shown in the drawings. Volatile storage media include dynamic memory, such as main memory of such a computer platform. Tangible transmission media include coaxial cables; copper wire and fiber optics, including the wires that comprise a bus within a computer system. Carrier-wave transmission media may take the form of electric or electromagnetic signals, or acoustic or light waves such as those generated during radio frequency (RF) and infrared (IR) data communications. Common forms of computer-readable media therefore include for example: a floppy disk, a flexible disk, hard disk, magnetic tape, any other magnetic medium, a CD-ROM, DVD or DVD-ROM, any other optical medium, punch cards paper tape, any other physical storage medium with patterns of holes, a RAM, a ROM, a PROM and EPROM, a FLASH-EPROM, any other memory chip or cartridge, a carrier wave transporting data or instructions, cables or links transporting such a carrier wave, or any other medium from which a computer may read programming code and/or data. Many of these forms of computer readable media may be involved in carrying one or more sequences of one or more instructions to a processor for execution.

The computer system 101 can include or be in communication with an electronic display 135 that comprises a user interface (UI) 140 for providing, for example, a result of a measuring of the device, an information about a sample, a link to a searchable database of reference values, or combination thereof. Examples of UI's include, without limitation, a graphical user interface (GUI) and web-based user interface.

Methods and systems of the present disclosure can be implemented by way of one or more algorithms. An algorithm can be implemented by way of software upon execution by the central processing unit 105. The algorithm can, for example, be a trained algorithm, trained by a plurality of training samples, such as blood samples having an absence or reduced level of one or more factors. An algorithm may be a supervised learning algorithm.

### EXAMPLES

### Example 1 - Instrument Test Protocol

Keyed tubes of aPTT/saline buffer and CaC12 solution will be inserted into correct ports on piercing adapter. The cartridge will be inserted into an instrument. The cartridge will be clamped. All solenoid valves will be turned on. A pressure pump will be turned on. Reagent tubes will be clamped. 40uL of whole blood will be pipetted into sample port. 10 uL of sample will be drawn into a metering channel @ 75 uL/s, it will be repeated 3 times with 5 seconds of waiting between each draw. A sample port will be covered. A script will be run-the following steps will be carried out autonomously: (a) Dispense 150 uL of aPTT/saline into control mixing chamber @ 75 uL/seconds; (b) Dispense 450 uL of aPTT/saline into Test mixing chamber @ 75 uL/seconds; (c) Stir test mixing chamber for 30 seconds; (d) Incubate for 4:30; (e) Dispense 150 uL of CaCl2 into control mixing chamber @ 75 uL/seconds; (f) Stir test mixing chamber for 5 seconds, start recording data, dispense 150 uL CaC12 into test mixing chamber @ 75 uL/s, continue mixing for an additional 5 seconds; (g) Draw 220 uL into test detection chamber @ 55 uL/s; and (h) Stop recording data after 3 minutes.

### Example 2 - Coagulation Time Measurements

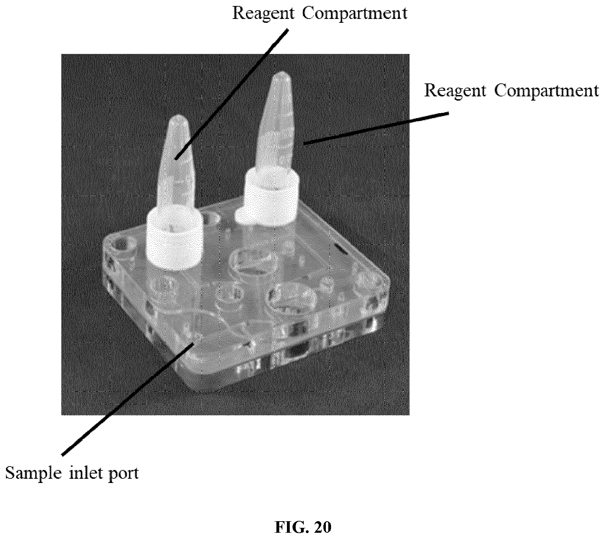

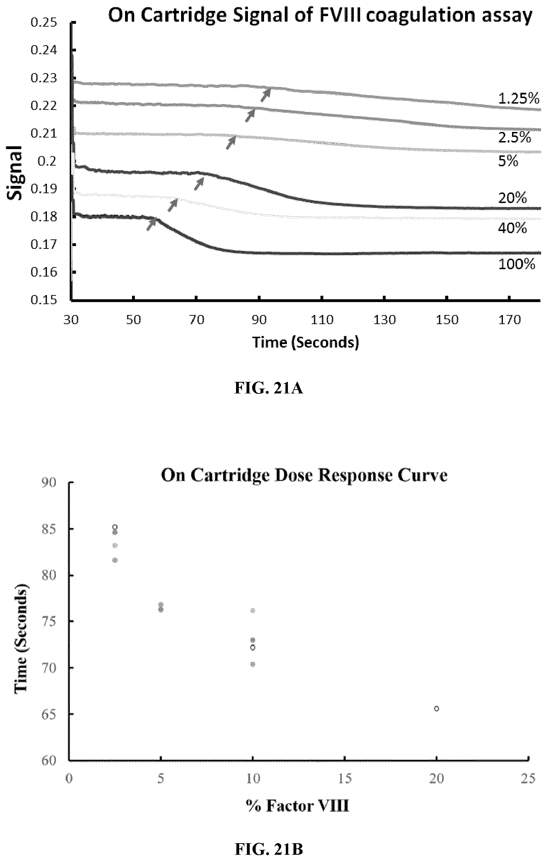

Referring to **FIG. 20** and **FIG. 21****,** after inserting a drop of blood (for example, a finger stick or a venous sample) onto a device, the user may insert the cartridge into a system, such as a compact instrument. The compact instrument may include a detector, such as an optical detector, a computer processor, a database, or any combination thereof. Sample processing and detection may be fully automated, and quantitative results may be provided within about 20 minutes, about 15 minutes, about 10 minutes, or about 5 minutes.

The one or more of the following assay steps may be integrated into the device: (a) user may insert a drop of whole blood (finger stick or venous sample) into the cartridge (from about 10 to about 29 µl volume); (b) about 10 µl of blood may be metered and mixed together with one or more reagents of the device (c) the mixture may be warmed to 37°C; (d) an aliquot of a calcium source (such as CaCl₂) may be added into the mixture to trigger a coagulation event; (e) at least a portion of the mixture may be pushed into a detection chamber; and (f) a coagulation may be detected, such as via optical detection. Additional assay steps may be included or downstream analysis, such as measuring a presence or absence of a co-factor or measuring a coagulation response of a control sample may also be included.

Referring to **FIG. 20****,** a sample can be provided to a device. The device can measure a factor (such as Factor VIII) coagulation time from a single drop of whole blood in about 15 minutes. In some cases, the device can contain two on-board wet reagents (such as shown in **FIG 20**) and lyophilized reagents together with control reagents. Multiple elastomeric valves can be included on the device to allow for fluidic control. The device or a portion thereof can be prototyped using a lamination approach. This approach may permit rapid prototyping and bonding of various materials such as plastics, elastomers, 3D printed parts, filters, or any combination thereof.

Referring to **FIG. 21A-B****,** results obtained from the device demonstrate that the coagulation time of the on-board processed sample may be dependent on the amount of factor concentration (such as Factor VIII concentration). As shown in **FIG. 21A****,** the detector response measured from detection chamber can demonstrate that the coagulation event can be detected and the shape of the response curve can be dependent on the Factor VIII concentration. **FIG. 21B** shows the coagulation time extracted from the data plotted against the spiked in Factor VIII concentration in the sample. Quantitative measurement of Factor VIII level with whole blood samples on the disposable cartridges can be demonstrated.

Referring to **FIG. 21A-B****,** cartridge test results are shown using 33% hematocrit blood with various adjusted concentrations of Factor VIII. Referring to **FIG. 21A****,** an optical response of the whole blood measured in the cartridge detection chamber can demonstrate a decrease in coagulation time as Factor VIII concentration dropped in the sample (indicated by arrow). Referring to **FIG. 21B****,** a dose response curve can be obtained on a cartridge at low Factor VIII concentration based on an algorithm to calculate the coagulation time for the sample in various cartridges.

Results may demonstrate a device that can quantitatively measure Factor VIII level in a single drop of whole blood. The test may be fully automated and provide a result in about: 20 minutes, 15 minutes, 10 minutes or less. Such a device or system can significantly simplify hemophilic disease diagnostics and management.

While preferred embodiments of the present invention have been shown and described herein, it will be obvious to those skilled in the art that such embodiments are provided by way of example only. It is not intended that the invention be limited by the specific examples provided within the specification. While the invention has been described with reference to the aforementioned specification, the descriptions and illustrations of the embodiments herein are not meant to be construed in a limiting sense. Numerous variations, changes, and substitutions will now occur to those skilled in the art without departing from the invention. Furthermore, it shall be understood that all aspects of the invention are not limited to the specific depictions, configurations or relative proportions set forth herein which depend upon a variety of conditions and variables. It should be understood that various alternatives to the embodiments of the invention described herein may be employed in practicing the invention. It is therefore contemplated that the invention shall also cover any such alternatives, modifications, variations or equivalents. It is intended that the following claims define the scope of the invention and that methods and structures within the scope of these claims and their equivalents be covered thereby.

## Claims

1. A method comprising:
(a) obtaining a blood sample from a subject;
(b) adding a portion of said blood sample to a solution to form a mixture,
wherein said solution comprises at least one Activated Partial Thromboplastin Time (aPTT) reagent, a salt solution, and a factor deficient plasma;
(c) adding a calcium salt to said mixture; and
(d) detecting coagulation of said blood sample.

2. The method of claim 1, wherein said blood sample is whole blood.

3. The method of claim 1, wherein said blood sample is blood plasma.

4. The method of claim 1, wherein said portion of said blood sample is a non-diluted portion.

5. The method of claim 1, wherein said factor deficient plasma is reconstituted.

6. The method of claim 1, wherein said factor deficient plasma is depleted of at least one of: Factor II, Factor V, Factor VII, Factor VIII, Factor IX, Factor X, Factor XI, or Factor XII.

7. The method of claim 1, wherein said factor deficient plasma is depleted of Factor VIII.

8. The method of claim 1, wherein said subject has or is suspected of having a clotting disorder.

9. The method of claim 8, wherein said clotting disorder is hemophilia.

10. The method of claim 1, wherein said salt solution contains saline.

11. The method of claim 1, wherein said aPTT reagent contains a silica acid, an ellagic acid, a phospholipid, or any combination thereof.

12. The method of claim 1, wherein said calcium salt contains calcium chloride, calcium acetate, calcium carbonate, calcium glubionate, calcium gluconate, calcium hydroxide, calcium nitrate, calcium sulfonate, calcium phosphate, or a mixture of any one of these.

13. The method of claim 1, wherein said calcium salt is a calcium chloride.

14. The method of claim 1, wherein said detecting employs an imaging system.

15. A cartridge comprising:
(a) a sample inlet port configured to receive a blood sample;
(b) a mixing compartment configured to receive at least a portion of said blood sample from said sample inlet port, wherein said mixing compartment contains a depleted plasma that is depleted of a coagulation factor, wherein upon contact of said portion of said blood sample with said depleted plasma, a mixture forms;
(c) a first reagent compartment that contains a calcium salt, wherein said mixing compartment is in fluidic communication with said first reagent compartment and is configured to receive a portion of said calcium salt from said first reagent compartment; and
(d) a detection compartment configured to receive at least a portion of said mixture from said mixing compartment.

16. The cartridge of claim 15, further comprising a second reagent compartment that contains a coagulation contact phase activator, wherein said mixing compartment is in fluidic communication with said second reagent compartment and is configured to receive a portion of said coagulation contact phase activator from said second reagent compartment.

17. The cartridge of claim 16, wherein said first reagent compartment or said second reagent compartment are removable from said cartridge.

18. The cartridge of claim 16, wherein said first reagent compartment or said second reagent compartment further contains a citrate source, a tissue factor, a phospholipid, or any combination thereof.

19. The cartridge of claim 16, further comprising a capillary, wherein said capillary is configured to fluidically connect said mixing compartment with said first reagent compartment or said second reagent compartment.

20. The cartridge of claim 15, wherein said depleted plasma is lyophilized.

21. The cartridge of claim 15, wherein said depleted plasma is depleted of at least one of: Factor II, Factor V, Factor VII, Factor VIII, Factor IX, Factor X, Factor XI, or Factor XII.

22. The cartridge of claim 15, further comprising a reference compartment, wherein said reference compartment contains a reference sample.

23. The cartridge of claim 22, wherein said reference sample is a plasma containing said coagulation factor.

24. The cartridge of claim 22, wherein said reference sample is lyophilized.

25. The cartridge of claim 22, wherein said reference compartment is in fluidic communication with said first reagent compartment and is configured to receive at least a portion of said calcium salt from said first reagent compartment.

26. The cartridge of claim 15, wherein said calcium salt comprises calcium chloride, calcium acetate, calcium carbonate, calcium glubionate, calcium gluconate, calcium hydroxide, calcium nitrate, calcium sulfonate, calcium phosphate, or a mixture of any one of these.

27. The cartridge of claim 16, wherein said coagulation contact phase activator is a kaolin.

28. The cartridge of claim 16, wherein said coagulation contact phase activator is an ellagic acid.

29. The cartridge of claim 15, wherein said detection compartment is configured to communicate with a detector.

30. A system comprising said cartridge of claim 15 and a detector.

31. The system of claim 30, wherein said detector is an imaging detector.

32. The system of claim 30, wherein said cartridge is configured to communicate with said detector.

33. A kit comprising said cartridge of claim 15, a sample collection tool, and instructions for use.

34. The kit of claim 33, further comprising an additional reagent compartment provided separately from said cartridge, said additional reagent compartment comprising said calcium salt, said coagulation contact phase activator, or a combination thereof.

35. A method comprising:
(a) inputting a blood sample into a mixing compartment of a device;
(b) mixing at least a portion of said blood sample with a depleted plasma that is depleted of a coagulation factor to form a mixture in said mixing compartment;
(c) adding at least a portion of a citrate source, a coagulation contact phase activator, a tissue factor, a calcium salt, a phospholipid, or any combination thereof to said mixture; and
(d) transferring at least a portion of said mixture to a detection compartment; and
(e) detecting coagulation of said portion of said blood sample in said detection compartment.

36. The method of claim 35, wherein said detecting employs an imaging-based detector.

37. The method of claim 35, wherein said blood sample is whole blood.

38. The method of claim 35, further comprising detecting coagulation of a reference sample in a reference compartment.

39. The method of claim 35, wherein said coagulation contact phase activator and said calcium salt are sequentially added to said mixing compartment.

40. The method of claim 35, wherein said depleted plasma is depleted of at least one of: Factor II, Factor V, Factor VII, Factor VIII, Factor IX, Factor X, Factor XI, or Factor XII.
